# FASCICULE DE BREVET EUROPEEN

(11) **EP 3 271 369 B1**
(45) Date de publication et mention de la délivrance du brevet: **28.04.2021**
(21) Numéro de dépôt: 16714489.8
(22) Date de dépôt: 16.03.2016
(51) Int. Cl.: C07H 15/04, B01F 17/00

(54) **PROCÉDÉ D'OBTENTION DE DÉRIVÉS ET DE COMPOSITIONS À BASE D'ACIDE D-GALACTURONIQUE DIRECTEMENT À PARTIR DE PECTINES**
VERFAHREN ZUR HERSTELLUNG VON DERIVATEN UND ZUSAMMENSETZUNGEN AUF GALACTURONSÄURE-BASIS DIREKT AUS PEKTINEN
PROCESS FOR OBTAINING DERIVATIVES AND COMPOSITIONS BASED ON D-GALACTURONIC ACID DIRECTLY FROM PECTINS

(30) Priorité: 16.03.2015 FR 1552119
(43) Date de publication de la demande: 24.01.2018
(73) Titulaire: École Nationale Supérieure De Chimie, 35000 Rennes (FR); Conseil Départemental d'Ille-et-Vilaine, 35000 Rennes (FR)
(72) Inventeur: MILLIASSEAU, Damien, 92250 La Garenne-Colombes (FR); BENVEGNU, Thierry, 35000 Rennes (FR); JEFTIC, Jelena, 35700 Rennes (FR); PLUSQUELLEC, Daniel, 35230 Noyal-châtillon-sur-Seiche (FR)
(74) Mandataire: Novagraaf Technologies
(86) Numéro de dépôt international: PCT/FR2016/050578
(87) Numéro de publication internationale: WO 2016/146941

(56) Documents cités:
- EP-A1- 0 635 516
- FR-A1- 2 840 306
- US-A1- 2009 186 833
- MYRIAM ROUSSEL ET AL.: "Synthesis and Physico-Chemical Properties of Novel Biocompatible Alkyl D-Mannopyranosiduronate Surfactants Derived from Alginate", EUR. J. ORG. CHEM., 2005, pages 3085-3094, XP002748888, DOI: 10.1002/ejoc.20050026
- GARNA H ET AL: "Kinetic of the hydrolysis of pectin galacturonic acid chains and quantification by ionic chromatography", FOOD CHEMISTRY, ELSEVIER LTD, NL, vol. 96, no. 3, 1 juin 2006 (2006-06-01), pages 477-484, XP027989399, ISSN: 0308-8146 [extrait le 2006-06-01]
- DATABASE CAPLUS [Online] CHEMICAL ABSTRACTS SERVICE, COLUMBUS, OHIO, US; XIE, WENQUAN ET AL: "Catalyst and method for selectively oxidizing primary hydroxy of protected monosaccharide", retrieved from STN Database accession no. 2008:297635 & XIE, WENQUAN ET AL: "Catalyst and method for selectively oxidizing primary hydroxy of protected monosaccharide", FAMING ZHUANLI SHENQING, 9PP. CODEN: CNXXEV, 2008,
- DATABASE CAPLUS [Online] CHEMICAL ABSTRACTS SERVICE, COLUMBUS, OHIO, US; MILKEREIT, G. ET AL: "Thermotropic and lyotropic properties of long chain alkyl glycopyranosides Part III: pH-sensitive headgroups", retrieved from STN Database accession no. 2004:8732 & MILKEREIT, G. ET AL: "Thermotropic and lyotropic properties of long chain alkyl glycopyranosides Part III: pH-sensitive headgroups", CHEMISTRY AND PHYSICS OF LIPIDS , 127(1), 47-63 CODEN: CPLIA4; ISSN: 0009-3084, 2004, DOI: 10.1016/J.CHEMPHYSLIP.2003.09.007 10.1016/J.CHEMPHYSLIP.2003.09.007

## Description

### Domaine technique

La présente invention se rapporte à un nouveau procédé d'obtention de dérivés et de compositions à base d'acide D-galacturonique directement à partir de pectines.

La présente invention trouve par exemple des applications dans les agents moussants, les solubilisants, les mouillants, les émulsifiants les détergents, notamment pour la cosmétologie, le domaine phytosanitaire et la détergence.

Dans la description ci-dessous, les références entre crochets ([]) renvoient à la liste des références présentée à la fin du texte.

### Etat de la technique

Les pectines figurent parmi les macromolécules les plus complexes présentes dans la nature. Bien que les études structurales aient fait l'objet de nombreux travaux récents (Perez et al., Biochimie, 85: 109-121, 2003; Voragen et al., Struct. Chem., 20 : 263-275, 2009) [1, 2], celles-ci requièrent encore des études complémentaires.

Les pectines sont des polysaccharides complexes abondants dans la nature car présents dans la paroi cellulaire de la majorité des végétaux terrestres et de quelques espèces d'algues dont elles assurent la rigidité. Outre les sucres neutres (L-rhamnose, D-galactose, L-arabinose, apiose, etc...), les pectines constituent actuellement la principale source connue d'acide D-galacturonique.

L'hydrolyse des pectines, en vue d'obtenir l'acide D-galacturonique sous forme de monomères ou d'oligomères, a fait l'objet de nombreux travaux. Des traitements chimiques, physiques ou enzymatiques ont été envisagés seuls ou en association pour dépolymériser les pectines.

Les hydrolyses par voie acide (Demande internationale WO 2009/004153 ; Thibault et al., Carbohydr. Res., 238 : 271-286, 1993 ; Diaz et al., J. Agric. Food Chem., 55 : 5131-5136, 2007 ; Garna et al., J. Agric. Food Chem., 52 : 4562-4659, 2004) [3-6] permettent la désacétylation et l'hydrolyse des esters ainsi que l'élimination des sucres neutres (L-rhamnose, L-arabinose, D-glucose, D-xylose, D-galactose, etc...) mais les liaisons glycosidiques α-(1-4) entre unités galacturoniques sont plus résistantes, et les réactions aboutissent de ce fait à des oligo-galacturonates.

Les pectinases sont un groupe hétérogène d'enzymes qui sont susceptibles : soit d'hydrolyser les fonctions esters méthyliques fréquentes sur les unités galacturoniques, soit d'éliminer les sucres neutres des régions « hérissées » des pectines (rhamnogalacturonases, arabinases, galactanases, etc...), soit de dépolymériser les chaines galacturoniques (Combo et al., Biotechnol. Agron. Soc. Environ., 15 : 153-164, 2011) [7]. Les mécanismes de rupture des chaines polygalacturoniques sont susceptibles d'intervenir par deux voies principales : i) l'hydrolyse des liaisons glycosidiques α-(1-4) des acides polygalacturoniques sous l'action des poly(méthyl)galacturonases, conduisant à des oligo-galacturonates et, ii) la rupture des liaisons glycosidiques par des mécanismes de β-éliminations conduisant à des uronides insaturés à l'extrémité non réductrice des oligomères galacturoniques. De ce fait, les préparations enzymatiques commerciales ne permettent pas d'envisager l'obtention de structures parfaitement définies (Demande JP 2012187042 ; Demande EP 2308989 ; Demande KR 20130070396 ; Demande UA 54330 ; Demande GB 792518 ; Demande US 2014134310) [8-13].

Les pectines, les oligo-galacturonates issus de l'hydrolyse partielle du polymère naturel et l'acide D-galacturonique issu de l'hydrolyse complète du polymère, font déjà l'objet de diverses applications bien maitrisées ou émergentes. Le domaine alimentaire constitue actuellement le principal utilisateur de pectines ou dérivés en tant qu'agents de texture, gélifiants, épaississants et stabilisants (Laurent et Boulenguer, Food Hydrocoll., 17 : 445-454, 2003 ; Thakur et al., Crit. Rev. Food Sci. Nutr., 37 : 47-73, 1997 ; Mesbahi et al., Food Hydrocoll., 19 : 731-738, 2005) [14-16]. Des travaux récents mettent par ailleurs en évidence l'intérêt de fragments pectiques comme ingrédients alimentaires notamment dans le secteur des prébiotiques (Courtois, Curr. Opin. Microbiol., 12 : 261-273, 2009 ; Wang, Food Res. Int., 42 : 8-12, 2009) [17, 18]. De nombreux exemples mettent également en évidence les propriétés immunomodulatrices remarquables de ces poly- et oligosaccharides ainsi que l'aptitude des acides pectiques à interagir avec divers effecteurs des systèmes immunologiques. Ceci peut d'ailleurs expliquer les effets bénéfiques des plantes médicinales utilisées en médecine traditionnelle. Ce domaine particulier a fait l'objet de revues récentes (Paulsen et Barsett, Adv. Polym. Sci., 186 : 69-101, 2005 ; Yamada et Kiyohara, Comprehensive Glycoscience, Elsevier, pp. 663-694, 2007 ; Chlubnova et al., Nat. Prod. Rep., 28 : 937-952, 2011) [19-21] ainsi que de divers Brevets (KR 20130070396 ; EP 2308989 ; KR 20130070526 ; US 3316242 ; JP 2007254403) [22-26]. Les mécanismes restent cependant à élucider.

De par leurs structures moléculaires, les acides pectiques et l'acide D-galacturonique constituent d'excellents agents complexants de cations métalliques (Novosel'skaya et al., Chemistry of Natural Compounds, 36 : 1-10, 2000) [27].

Enfin, l'acide D-galacturonique sous forme de monomère, issu de l'hydrolyse des pectines, a fait l'objet de divers travaux dans le laboratoire des inventeurs et dans l'industrie comme source de têtes polaires pour des molécules tensioactives (Bertho et al., J. Chem. Soc. Chem. Commun., 1391-1393, 1995 ; Auvray et al., J. Mater. Chem., 7 : 1373-1376, 1997 ; Ferrières et al., Carbohydr. Res., 311 : 15-25, 1998 ; Benvegnu et al., Polym. Int., 52 : 500-506, 2003 ; Goodby et al., Chem. Soc. Rev., 36 : 1971-2032, 2007 ; Demande Internationale WO 9302092 ; Demande FR 9402771) [28-34]. Les inventeurs ont ainsi mis au point des synthèses originales de tensioactifs bi- ou monocaténaires, neutres ou anioniques à partir du monomère « acide D-galacturonique » dans lesquels les phénomènes de tautomérie et d'anomérie ont été maitrisés pour aboutir à des tensioactifs de structures parfaitement définies. L'étude des propriétés interfaciales a mis en évidence que le procédé de l'invention permet d'aboutir à des composés hydrotropes, détergents ou émulsifiants. Par exemple la Demande Internationale WO 9302092 décrit des dérivés de l'acide D-galacturonique, leurs applications notamment comme agents tensioactifs non ioniques ou anioniques et leurs procédés de préparation à partir du monomère commercial de l'acide D-galacturonique préalablement purifié. Cet acide est obtenu industriellement via une série de traitements longs et couteux à mettre en œuvre (hydrolyse totale des pectines ; élimination des sucres neutres résultant de cette hydrolyse ; mise en œuvre d'une purification de l'acide D-galacturonique sous forme de sels de cations métalliques (Na⁺, K+, etc...) sur une résine échangeuse d'ions ; acidification de la solution aqueuse ainsi obtenue notamment par des hydracides tels que l'acide chlorhydrique, l'acide sulfurique, etc... ; isolement de l'acide D-galacturonique notamment par cristallisation). D'ailleurs les tensioactifs dérivés de l'acide D-galacturonique décrits dans la Demande Internationale WO 9302092 n'ont connu à ce jour aucun développement industriel, le coût de l'obtention de l'acide sous forme de monomère purifié étant prohibitif.

De ce fait, ces techniques ne répondent pas à la demande de tensioactifs neutres (bicaténaires) ou anioniques (monocaténaires) pouvant être préparés à des coûts concurrentiels et d'une efficacité plus grande que leurs homologues issus de la pétrochimie.

Il existe donc un réel besoin d'un nouveau procédé de synthèse de dérivés et compositions à base d'acide D-galacturonique palliant les défauts, inconvénients et obstacles de l'art antérieur, en particulier d'un procédé permettant de maîtriser l'obtention à l'échelle industrielle de dérivés et compositions d'usage de l'acide D-galacturonique, de réduire les coûts, d'améliorer les propriétés attendues des dérivés obtenus dans le domaine des tensioactifs, et répondant aux principes de la « chimie verte ».

### Description de l'invention

Bien qu'il ait été récemment bien établi qu'il est difficile de maîtriser l'hydrolyse complète des pectines (Wikiera et al., Food Chem., 172 : 675-680, 2015 (EPub 2014)) [35], bien que la liaison entre unités galacturoniques soit considérée comme plus solide que les autres liaisons glycosidiques des pectines impliquant les sucres neutres, les Inventeurs ont réussi à mettre au point un nouveau procédé permettant d'aboutir à la réalisation soit de synthons soit de compositions dérivées de l'acide D-galacturonique ou des produits d'usage (composés hydrotropes, détergents non ioniques ou anioniques, agents émulsifiants, etc....), directement à partir de pectines, en mettant en œuvre à la fois l'étape d'hydrolyse et l'étape de glycosylation / transglycosylation / estérification / transestérification ; ce qui est encore plus difficile à maitriser.

La présente invention a donc pour objet un procédé de préparation de compositions comprenant
(i) des dérivés de l'acide D-galacturonique de formule (I) : 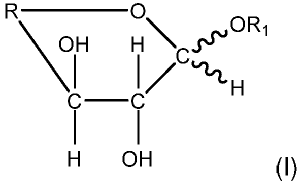 où
   - R₁ est une chaine de 2 à 22 atomes de carbone linéaire ou ramifiée, saturée ou insaturée ;
   - R est CH-CH(OH)-CO₂R₂ ou CH(OH)-CH-CO₂R₂, dont le carbone ne portant pas le groupe hydroxyle est lié à l'atome d'oxygène endocyclique ;
   - R₂ est un hydrogène, R₁, un atome de métal alcalin, un atome de métal alcalino-terreux, ou un groupe ammonium quaternaire de formule (II) : 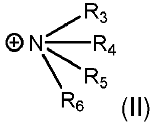
   où chacun des R₃ à R₆ est indépendamment un atome d'hydrogène, un alkyle ayant de 1 à 6 atomes de carbone ou un hydroxyalkyle ayant de 1 à 6 atomes de carbone, et
(ii) de 5 à 30% en masse de glycosides d'alkyle dérivés de sucres neutres ;
   et caractérisé en ce que ledit procédé one pot comprend :
   a) une étape d'hydrolyse acide complète d'une pectine ;
   b) une étape d'estérification et glycosylation de l'hydrolysat issu de l'étape
   a) par un alcool de formule R'OH ayant de 1 à 4 atomes de carbone ;
   c) une étape de trans-estérification et trans-glycosylation de l'alkyl galactoside uronate d'alkyl ou composé bicaténaire issu de l'étape b) par un alcool de formule R"OH ayant de 2 à 22 atomes de carbone où R'' est R₁ ;
   d) et optionnellement une étape de saponification de l'ester issu de l'étape c).

Les compositions finales contiennent entre 5 et 30 % en masse, de préférence 5 % en masse, de glycosides d'alkyles issus de dérivés des sucres neutres (L-rhamnose, L-arabinose, D-glucose, D-xylose, D-galactose, etc.) présents dans les pectines naturelles et résultant des réactions de glycosylation et de transglycosylation.

Par « pectine » au sens de la présente invention, on entend par exemple des pectines naturelles, telles que les pectines hautement méthylées, les pectines faiblement méthylées, les pectines amidées. De préférence, il s'agit de pectines faiblement méthylées.

Selon un mode de réalisation particulier de la présente invention, l'étape d'hydrolyse acide complète a) d'une pectine est réalisée en présence i) d'eau et/ou d'un solvant ionique tels que par exemple le chlorure de 1-butyl-3-méthylimidazolium [BMIM]CI, le bromure de 1-butyl-3-méthylimidazolium [BMIM]Br, le méthylsulfate de tris-(2-hydroxyéthyl)méthylammonium (HEMA) et l'acétate de 1-éthyl-3-méthylimidazolium [EMIM]AcO ; ledit solvant ionique comprenant typiquement jusqu'à 10% d'eau, et ii) d'un catalyseur acide, tels que par exemple l'acide chlorhydrique, l'acide sulfurique, un acide alkyl sulfurique tels que par exemple l'acide décyl ou lauryl sulfurique, un acide sulfonique tels que l'acide benzène sulfonique, l'acide paratoluène sulfonique, l'acide camphosulfonique, un acide alkylsulfonique tels que l'acide méthylsulfonique, l'acide décylsulfonique, l'acide laurylsulfonique, l'acide sulfosuccinique ou un sulfosuccinate d'alkyl tels que le sulfosuccinate de décyle ou sulfosuccinate de lauryle, les acides perhalohydriques, tel que l'acide perchlorique, des métaux tels que le fer, leurs oxydes ou leurs sels comme leurs halogénures. De préférence, il s'agit d'acide alkylsulfonique ou d'acide méthylsulfonique. Cette étape d'hydrolyse est réalisée, par exemple, en mettant en présence un équivalent de pectine ou de poly- ou oligo-galacturonate, 50 à 1000 équivalents d'eau et/ou de solvant ionique, et 10⁻³ à 10 équivalents de catalyseur acide, de préférence de 1,1 à 10 équivalents molaires d'acide alkylsulfonique et plus particulièrement de 2,5 équivalents molaires d'acide méthylsulfonique. Cette étape d'hydrolyse est préférentiellement réalisée entre 80°C et 120°C en autoclave, et de préférence portée au reflux de l'eau à la pression atmosphérique sur un temps de réaction pouvant être de 1 à 24 heures et de préférence sur un temps de réaction de 5 heures.

Selon un mode de réalisation particulier de la présente invention, l'étape d'estérification et glycosylation b) est réalisée sur le milieu réactionnel (hydrolysat) refroidi issu de l'étape a), et sans aucun traitement dudit milieu, en présence d'un alcool de formule R'OH, linéaire ou ramifié, saturé ou insaturé, où R' est composé de 1 à 4 atomes de carbone. De préférence l'alcool R'OH est le n-butanol. Cette étape d'estérification et glycosylation b) est réalisée, par exemple, en introduisant dans le milieu réactionnel 2 à 100 équivalents molaires d'alcool R'OH tel que défini ci-dessus et de préférence 100 équivalents molaires. Le montage est modifié avec l'ajout d'un Dean Stark permettant l'élimination de l'eau. La réaction est alors conduite au reflux de l'eau ou de l'azéotrope formé avec l'alcool R'OH, à pression atmosphérique, le temps que la quasi-totalité de l'eau soit éliminée. Puis la température est abaissée, de préférence à 80°C, et la réaction est poursuivie, de préférence sur 24 heures. La composition ainsi formée est constituée majoritairement d'un composé bicaténaire dérivé de l'acide D-galacturonique.

Selon un mode de réalisation préférée de la présente invention, la préparation d'alkyl galactoside uronate d'alkyle, où la chaine alkyle est plus longue, se poursuit par une étape de trans-estérification et trans-glycosylation c) réalisée sur cette composition constituée majoritairement du composé bicaténaire issue de l'étape b), en présence d'un alcool de formule R"OH, linéaire ou ramifié, saturé ou insaturé où R'' est composé de 2 à 22 atomes de carbone et est R₁. Par exemple, l'alcool R"OH est choisi dans le groupe constitué des alcools gras linéaires, saturés ou insaturés, tel que l'alcool oléique. Cette étape de trans-estérification et trans-glycosylation est réalisée, par exemple, en introduisant dans la composition 2 à 50 équivalents molaires d'alcool R"OH tel que défini ci-dessus, et de préférence de 4 équivalents molaires. Le montage n'est pas modifié, permettant de ce fait d'effectuer les réactions de trans-estérification et trans-glycosylation et de recycler l'alcool R'OH à chaîne courte, par exemple le n-butanol, préalablement utilisé pour la formation de la composition riche en composé bicaténaire dérivé de l'acide D-galacturonique, par exemple le (n-butyl-D-galactoside) uronate de n-butyle. La réaction de trans-estérification et trans-glycosylation c) est préférentiellement conduite à une température de préférence de 70°C et sous une pression réduite pour le recyclage de l'alcool R'OH précédemment cité. La composition ainsi formée constitue un produit d'usage dérivé de l'acide D-galacturonique tel qu'un agent hydrotrope, un détergent non-ionique ou un agent émulsifiant.

La présente invention a également pour objet un procédé de préparation de sels d'acides alkyl galactoside uroniques, ledit procédé comprenant une réaction de saponification d) de l'ester issu de l'étape de trans-estérification et trans-glycosylation c) en présence d'une base de formule M(OH)ₓ dans laquelle M est un métal alcalin ou alcalino-terreux, et x est la valence, ou d'une base de formule 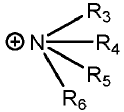 où chacun des R₃ à R₆ a la même définition que précédemment. De préférence la base est choisie dans le groupe constitué de : l'hydroxyde de sodium, l'hydroxyde de potassium, l'ammoniaque ou un hydroxyde d'alkyl(hydroxyalkyl) ammonium tel que par exemple l'hydroxyde de tris-(2-hydroxyéthyl)méthylammonium. Cette étape de saponification d) de l'ester est réalisée, par exemple, en mettant en présence un équivalent d'alkyl galactoside uronate d'alkyle avec 0,5-10 équivalents de base M(OH)ₓ telle que définie ci-dessus et de préférence de 1 à 3 équivalents. La réaction de saponification d) de l'ester est préférentiellement conduite à des températures comprises entre 0°C et 100°C, et de préférence entre 0°C et 60°C et pendant une durée de 15 minutes à plusieurs heures, et de préférence pendant une durée s'étalant sur toute une nuit. A l'issue de la réaction, tout ou partie de l'alcool R'OH, le solvant ionique ou le système de solvants ioniques utilisé peut éventuellement être éliminé par filtration ou par évaporation.

La présente invention a également pour objet un procédé de préparation d'acides alkyl galactoside uroniques, ledit procédé comprenant une réaction d'acidification des sels d'acides alkyl galactoside uroniques en présence d'un acide tels que l'acide chlorhydrique, l'acide sulfurique, un acide sulfonique ou une résine sulfonique sous sa forme H⁺. Cette réaction d'acidification est réalisée, par exemple, en mettant en présence un équivalent de sels d'acides alkyl galactoside uroniques avec un équivalent ou plus d'un acide tel que défini ci-dessus.

La présente invention a également pour objet une composition obtenue par un procédé selon l'invention, caractérisée en ce que ladite composition présente une teneur en glycosides d'alkyles dérivés de sucres neutres comprise entre 5 et 30 % en masse, de préférence d'environ 5 % en masse.

La présente invention a également pour objet l'utilisation des compositions de l'invention comme agent tensioactif, en particulier comme émulsifiants ou détergents.

Le procédé et les compositions qui font l'objet de la présente invention répondent au principe de la « chimie verte » : en utilisant exclusivement des matières premières biosourcées (pectines, alcool gras ou non) ou biocompatibles / biodégradables (acide méthane sulfonique et analogues) ; en mettant en œuvre une méthodologie sans groupements protecteurs et répondant ainsi au principe d'économie d'atomes ; en s'affranchissant de l'utilisation de solvants organiques, autres que les alcools réactifs, tant au niveau de la réaction que du traitement final du brut réactionnel ; en réalisant l'ensemble des réactions selon un procédé « one pot » pour aboutir directement à de nouvelles compositions tensioactives à partir de pectines sans isolement, ni purification des intermédiaires réactionnels ce qui permet d'éviter les pertes inévitables au cours des extractions d'où une amélioration des rendements, un gain de temps et un coût faible compatible avec un développement industriel rapide ; en recyclant les alcools à chaînes courtes (n-butanol, etc...) excédentaires ; en permettant d'aboutir ainsi aux composés dérivés et aux compositions à des prix concurrentiels par rapport au marché actuel ; et donc, en répondant aux enjeux économiques visés par les entreprises et les consommateurs. En outre il est à noter que les compositions obtenues par le procédé de l'invention sont riches en dérivés galacturoniques mais contiennent également des glycosides d'alkyle dérivés de sucres neutres représentatifs de la structure des pectines de départ (L-rhamnose, L-arabinose, D-glucose, D-galactose, D-xylose), entre 5 et 30% en masse, de préférence 5% en masse, qui modifient les propriétés physicochimiques des compositions finales et optimisent les performances tensioactives des compositions finales.

D'autres avantages pourront encore apparaître à l'homme du métier à la lecture des exemples ci-dessous, donnés à titre illustratif.

### Brève description des figures :

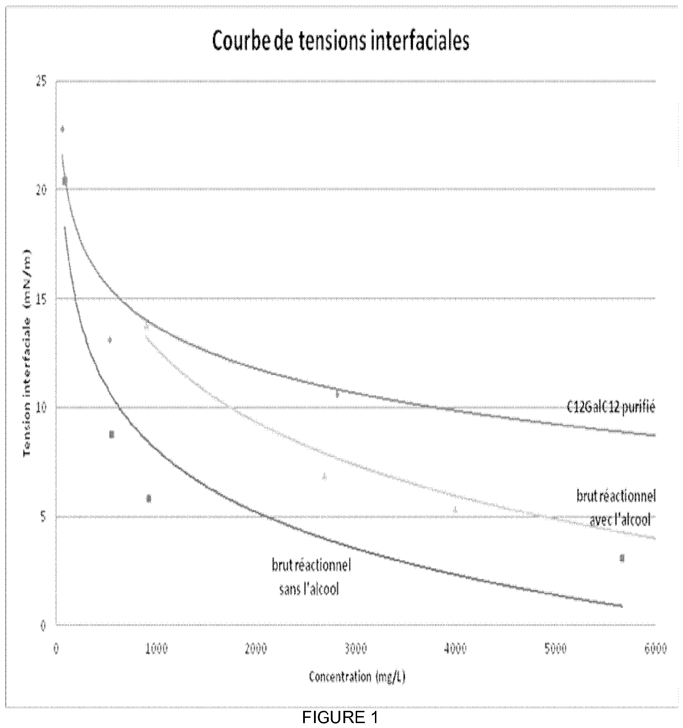

- La figure 1 représente les courbes de tensions interfaciales eau / huile végétale additionnées de C₁₂GalC₁₂, à l'état purifié ou sous forme de bruts réactionnels avec et sans alcool laurique issus du procédé de l'invention.

### EXEMPLES

### Exemple 1 : Préparation de compositions contenant majoritairement le (n-butyl-D-galactoside) uronate de n-butyle

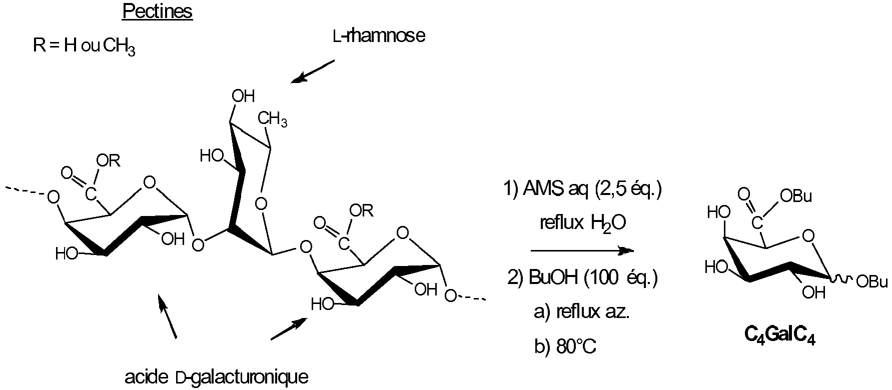

Dans un ballon monocol, 0,53 g de pectines faiblement méthylées 27NA/C (Cargill) (2,7 mmol, 1 éq.) ont été introduits. Environ 50 ml d'eau distillée (1000 éq.) ont été ajoutés, puis 0,63 ml d'AMS technique à 70 % (8,9 mmol, soit 2,5 éq. d'AMS pur).

La réaction a été portée au reflux de l'eau pendant 5 heures à pression atmosphérique pour favoriser l'hydrolyse en milieu acide.

Une fois le milieu refroidi, ont été ajoutés 25,0 ml de butan-1-ol (270 mmol, 100 éq.) et un Dean Stark au montage qui a permis d'éliminer l'eau. La réaction a alors été conduite au reflux de l'azéotrope à pression atmosphérique le temps que la quasi-totalité de l'eau soit éliminée puis la température a été abaissée à 80 °C et la réaction a été poursuivie sur 24h *(C₄GalC₄).*

Une fois la réaction terminée, le milieu réactionnel a été neutralisé par une solution aqueuse de soude puis évaporé à sec.

### Ce produit a constitué une composition aux propriétés d'usage recherchées.

Après chromatographie éventuelle sur gel de silice (Geduran® SI 60, systèmes d'éluants ; CH₂Cl₂ pur, CH₂Cl₂/AcOEt (4/1, v/v), CH₂Cl₂/AcOEt (1/1, v/v)), a été déterminée la présence de 0,50 g (1,6 mmol, 60 % de rendement) d'un mélange des formes isomères de (n-butyl-D-galactoside) uronate de *n-*butyle et d'environ 5 % d'un mélange constitué majoritairement de L-rhamnoside et D-galactoside de n-butyle.

### Exemple 2 : Préparation de compositions contenant majoritairement le [(Z)-octadec-9-enyl-D-galactoside] uronate de (Z)-octadec-9-enyle

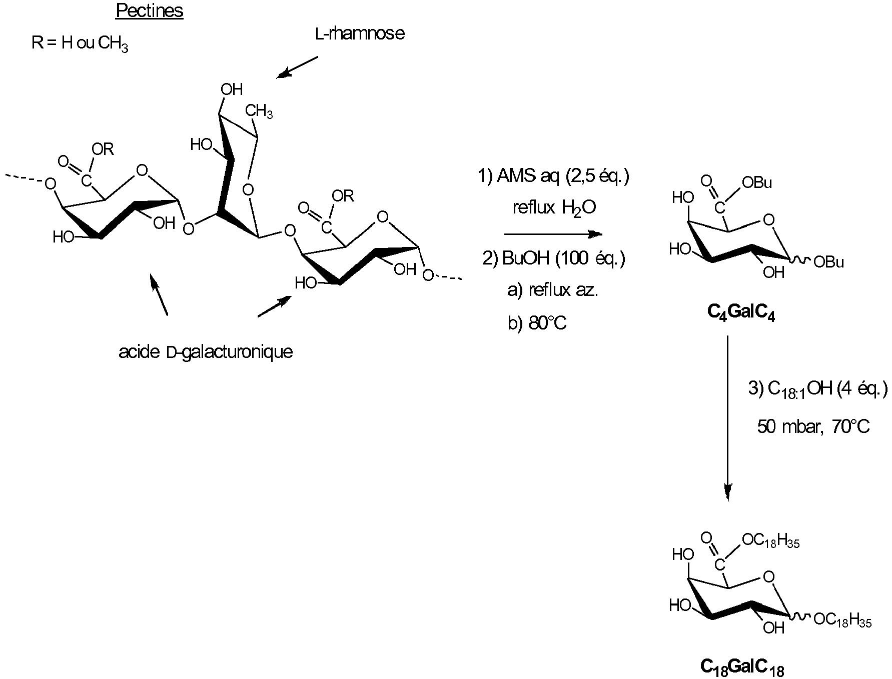

Dans un ballon monocol, 0,50 g de pectines faiblement méthylées 27NA/C (Cargill) (2,6 mmol, 1 éq.) ont été introduits. Environ 10 ml d'eau distillée (200 éq.) ont été ajoutés, puis 0,60 ml d'AMS technique à 70 % (8,4 mmol, soit 2,5 éq. d'AMS pur).

La réaction a été portée au reflux de l'eau pendant 5 heures à pression atmosphérique pour favoriser l'hydrolyse en milieu acide.

Une fois le milieu refroidi, ont été ajoutés 23,7 ml de butan-1-ol (260 mmol, 100 éq.) et un Dean Stark au montage qui a permis d'éliminer l'eau. La réaction a alors été conduite au reflux de l'azéotrope à pression atmosphérique le temps que la quasi-totalité de l'eau soit éliminée puis la température a été abaissée à 80 °C et la réaction a été poursuivie sur 24h *(C₄GalC₄).*

3,3 ml d'alcool oléique (10,4 mmol, 4 éq.) ont été ajoutés sans modifier le montage ce qui a permis d'effectuer la transestérification / transglycosylation tout en recyclant le butan-1-ol. Cette réaction a été conduite à 70°C sous pression réduite (jusqu'à 50 mbar) pendant 5 heures *(C₁₈GalC₁₈).*

Une fois la réaction terminée, le milieu réactionnel a été neutralisé par une solution aqueuse de soude puis évaporé à sec.

### Ce produit a constitué une composition aux propriétés d'usage recherchées.

Après chromatographie éventuelle sur gel de silice (Geduran® SI 60, systèmes d'éluants ; CH₂Cl₂ pur, CH₂Cl₂/AcOEt (8/2, v/v), CH₂Cl₂/AcOEt (1/1, v/v)), a été déterminée la présence de 0,72 g (1,04 mmol, 40 % de rendement) d'un mélange des quatre formes isomères de [(Z)-octadec-9-enyl-D-galactoside] uronate de (Z)-octadec-9-enyle et d'environ 5 % d'un mélange constitué majoritairement de L-rhamnoside et D-galactoside de (Z)-octadec-9-enyle.

### Exemple 3 : Préparation de compositions contenant majoritairement le sel de sodium de l'acide (lauryl-D-galactoside) uronique

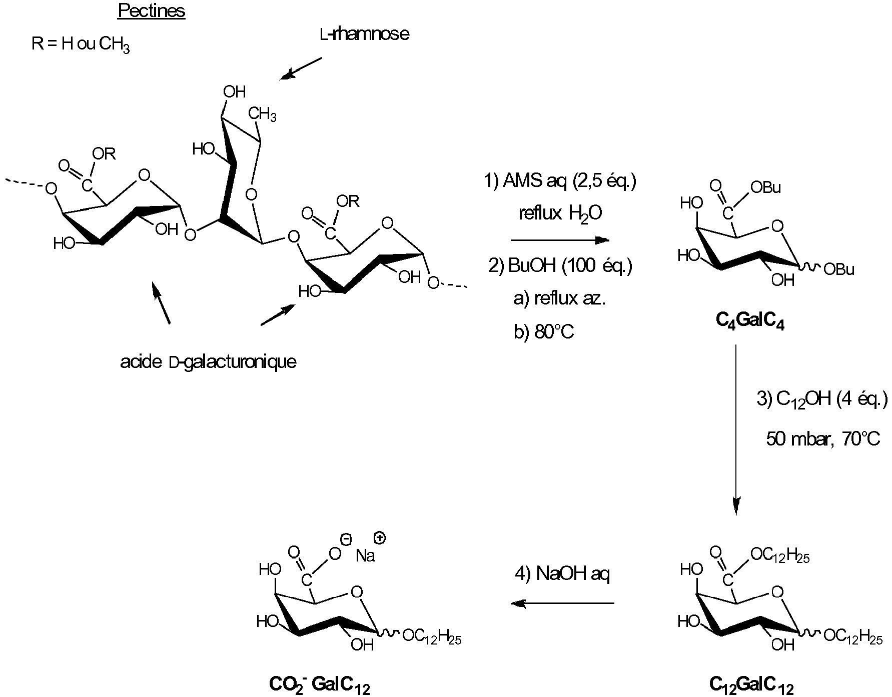

Dans un ballon monocol, 0,49 g de pectines faiblement méthylées 27NA/C (Cargill) (2,5 mmol, 1 éq.) ont été ajoutés. Environ 10 ml d'eau distillée (200 éq.) ont été ajoutés, puis 0,59 ml d'AMS technique à 70 % (8,3 mmol, soit 2,5 éq. d'AMS pur).

La réaction a été portée au reflux de l'eau pendant 5 heures à pression atmosphérique pour favoriser l'hydrolyse en milieu acide.

Une fois le milieu refroidi, ont été ajoutés 23,5 ml de butan-1-ol (255 mmol, 100 éq.) et un Dean Stark au montage qui a permis d'éliminer l'eau. La réaction a alors été conduite au reflux de l'azéotrope à pression atmosphérique le temps que la quasi-totalité de l'eau soit éliminée puis la température a été abaissée à 80 °C et la réaction a été poursuivie sur 24h *(C₄GalC₄).*

Ensuite 2,3 ml d'alcool laurique (10,3 mmol, 4 éq.) ont été ajoutés sans modifier le montage ce qui a permis d'effectuer la transestérification / transglycosylation tout en recyclant le butan-1-ol. Cette réaction a été conduite à 70°C sous pression réduite (jusqu'à 50 mbar) pendant 5 heures *(C₁₂GalC₁₂*).

Une fois la réaction terminée, environ 10 ml d'une solution aqueuse de soude 3N ont été ajoutés au milieu réactionnel provoquant ainsi la saponification de l'ester laurique. Cette étape a été laissée sous agitation à 30°C toute une nuit. Le milieu a ensuite été évaporé à sec *(CO₂⁻GalC₁₂).*

### Ce produit a constitué une composition aux propriétés d'usage recherchées.

Après chromatographie éventuelle sur gel de silice (Geduran® SI 60, systèmes d'éluants ; CH₂Cl₂ pur, CH₂Cl₂/AcOEt (8/2, v/v), CH₂Cl₂/AcOEt (1/1, v/v)), a été déterminée la présence de 0,37 g (1,02 mmol, 40 % de rendement) d'un mélange des formes isomères de (lauryl-D-galactoside) uronate de sodium et d'environ 5 % d'un mélange constitué majoritairement de L-rhamnoside et D-galactoside de lauryle.

A titre d'exemple, une étude comparative des propriétés superficielles du composé CO₂ ⁻GalC₁₂ purifié ou sous forme de bruts réactionnels issus du présent procédé a été réalisée au tensiomètre à anneau (KRÜSS type K100C). Ces analyses de tensions superficielles ont été réalisées à 25°C avec de l'eau milliQ à laquelle a été additionné l'échantillon à analyser mis en solution à 1%. Les résultats sont récapitulés dans le tableau 1 ci-dessous :

**Tableau 1**

| **Composé mis en solution à 1%** | γCMC **(mN/m)** |
|---|---|
| CO₂⁻GalC₁₂ purifié | **35,8** |
| CO₂⁻GalC₁₂ brut | **24,2** |

Les résultats montrent bien que la présence de glycosides d'alkyles et d'alcool résiduel abaisse largement la tension superficielle par rapport au composé D-galacturonique purifié.

### Exemple 4 : Préparation de compositions contenant majoritairement l'acide [(Z)-octadec-9-enyle-D-galactoside] uronique

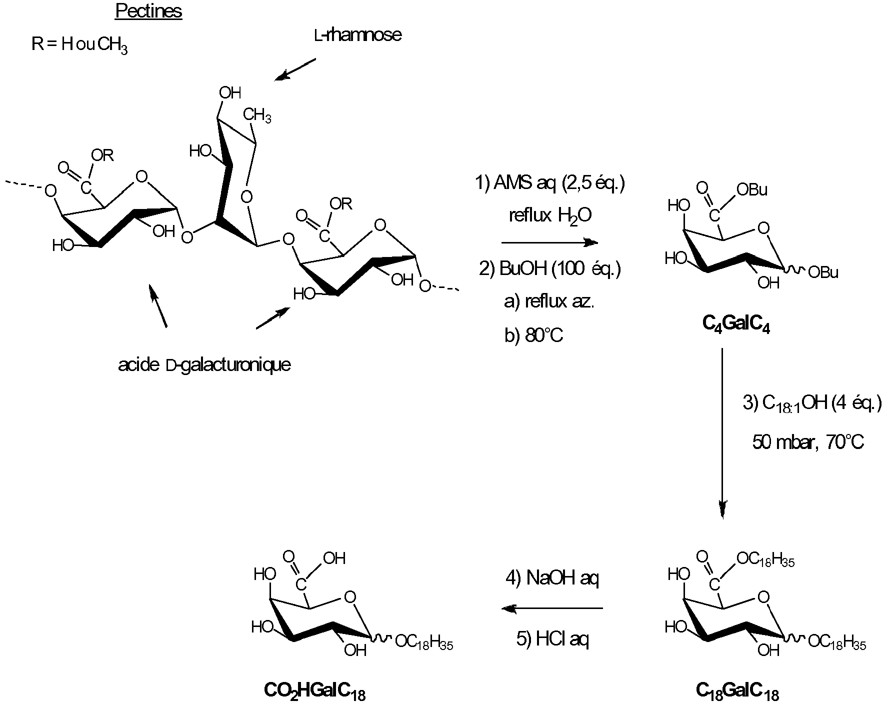

Dans un ballon monocol, 0,50 g de pectines faiblement méthylées 27NA/C (Cargill) (2,6 mmol, 1 éq.) ont été introduits. Environ 10 ml d'eau distillée (200 éq.) ont été ajoutés, puis 0,60 ml d'AMS technique à 70 % (8,4 mmol, soit 2,5 éq. d'AMS pur).

La réaction a été portée au reflux de l'eau pendant 5 heures à pression atmosphérique pour favoriser l'hydrolyse en milieu acide.

Une fois le milieu refroidi, ont été ajoutés 23,8 ml de butan-1-ol (260 mmol, 100 éq.) et un Dean Stark au montage qui a permis d'éliminer l'eau. La réaction a alors été conduite au reflux de l'azéotrope à pression atmosphérique le temps que la quasi-totalité de l'eau soit éliminée puis la température a été abaissée à 80 °C et la réaction a été poursuivie sur 24h *(C₄GalC₄).*

Ensuite 3,3 ml d'alcool oléique (10,4 mmol, 4 éq.) ont été ajoutés sans modifier le montage ce qui a permis d'effectuer la transestérification / transglycosylation tout en recyclant le butan-1-ol. Cette réaction a été conduite à 70°C sous pression réduite (jusqu'à 50 mbar) pendant 5 heures *(C₁₈GalC₁₈).*

Une fois la réaction terminée, environ 10 ml d'une solution aqueuse de soude 3N ont été ajoutés au milieu réactionnel provoquant ainsi la saponification de l'ester oléique. Cette étape a été laissée sous agitation à 40°C toute une nuit.

Une fois la saponification complète, une solution aqueuse d'acide chlorhydrique a été ajoutée jusqu'à atteindre un pH inférieur à 3. Le milieu a ensuite été évaporé à sec *(CO₂HGalC₁₈).*

### Ce produit a constitué une composition aux propriétés d'usage recherchées.

Après chromatographie éventuelle sur gel de silice (Geduran® SI 60, systèmes d'éluants ; CH₂Cl₂ pur, CH₂Cl₂/AcOEt (8/2, v/v), CH₂Cl₂/AcOEt (1/1, v/v)), a été déterminée la présence de 0,46 g (1,04 mmol, 40 % de rendement) d'un mélange des formes isomères de [(Z)-octadec-9-enyl-D-galactoside] uronique et d'environ 5 % d'un mélange constitué majoritairement de L-rhamnoside et D-galactoside de (Z)-octadec-9-enyle.

### Exemple 5 : Préparation de compositions contenant majoritairement le (lauryl-D-galactoside) uronate de lauryle

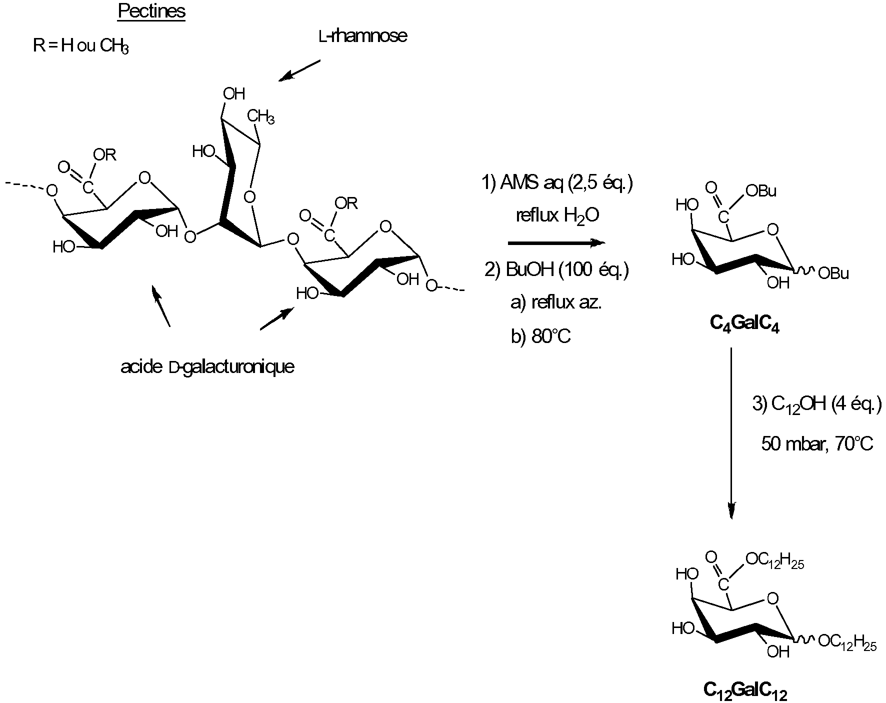

Dans un ballon monocol, 2,50 g de pectines faiblement méthylées 27NA/C (Cargill) (13,1 mmol, 1 éq.) ont été introduits. Environ 50 ml d'eau distillée (200 éq.) ont été ajoutés, puis 3,0 ml d'AMS technique à 70 % (42,5 mmol, soit 2,5 éq. d'AMS pur).

La réaction a été portée au reflux de l'eau pendant 5 heures à pression atmosphérique pour favoriser l'hydrolyse en milieu acide.

Une fois le milieu refroidi, ont été ajoutés 120 ml de butan-1-ol (1310 mmol, 100 éq.) et un Dean Stark au montage qui a permis d'éliminer l'eau. La réaction a alors été conduite au reflux de l'azéotrope à pression atmosphérique le temps que la quasi-totalité de l'eau soit éliminée puis la température a été abaissée à 80 °C et la réaction a été poursuivie sur 24h *(C₄GalC₄).*

11,9 ml d'alcool oléique (10,4 mmol, 4 éq.) ont été ajoutés sans modifier le montage ce qui a permis d'effectuer la transestérification / transglycosylation tout en recyclant le butan-1-ol. Cette réaction a été conduite à 70°C sous pression réduite (jusqu'à 50 mbar) pendant 5 heures *(C₁₂GalC₁₂)*.

Une fois la réaction terminée, le milieu réactionnel a été neutralisé par une solution aqueuse de soude puis évaporé à sec.

### Ce produit a constitué une composition aux propriétés d'usage recherchées.

Après chromatographie éventuelle sur gel de silice (Geduran® SI 60, systèmes d'éluants ; CH₂Cl₂ pur, CH₂Cl₂/AcOEt (8/2, v/v), CH₂Cl₂/AcOEt (1/1, v/v)), ont été déterminées les présences majoritaires de 7,2 g d'alcool laurique et de 2,8 g (5,3 mmol, 40 % de rendement) d'un mélange des quatre formes isomères de (lauryl-D-galactoside) uronate de lauryle, et d'environ 5 % d'un mélange constitué majoritairement de L-rhamnoside et D-galactoside de lauryle.

A titre d'exemple, une étude comparative des propriétés interfaciales eau / huile végétale additionnées de C₁₂GalC₁₂, à l'état purifié ou sous forme de bruts réactionnels issus du présent procédé, a été réalisée au tensiomètre à anneau (KRÜSS type K100C). Ces analyses de tensions interfaciales ont été réalisées à 25°C entre de l'eau déminéralisée et de l'huile de tournesol commerciale à laquelle a pu être additionnée l'échantillon à analyser. Les résultats sont récapitulés dans le tableau 2 ci-dessous et dans la figure 1.

**Tableau 2**

| **C₁₂GalC₁₂ purifié** | | **brut réactionnel sans l'alcool laurique** | | **brut réactionnel avec l'alcool laurique** | |
|---|---|---|---|---|---|
| **C (mg/L)** | y **(mN/m)** | C **(mg/L)** | γ **(mN/m)** | **C (mg/L)** | γ **(mN/m)** |
| 60 | 22,82 | 85,7 | 20,39 | 896,6 | 13,79 |
| 537,1 | 13,15 | 554,2 | 8,78 | 2683 | 6,92 |
| 2809 | 10,63 | 918,9 | 5,87 | 3994,6 | 5,39 |
| - | - | 5663,8 | 3,11 | - | - |

Les principales conclusions sont les suivantes :
- La tension interfaciale est plus faible pour les compositions de mélanges majoritairement constitués de (lauryl-D-galactoside) uronate de lauryle que pour ce même composé purifié ; ce qui prouve un effet bénéfique des co-produits de réactions.
- L'alcool gras résiduel ne limite pas l'efficacité à composition égale en tensioactifs du brut réactionnel.

### Liste des références

1. Perez et al., Biochimie, 85 : 109-121, 2003
2. Voragen et al., Struct. Chem., 20 : 263-275, 2009
3. Demande internationale WO 2009/004153
4. Thibault et al., Carbohydr. Res., 238 : 271-286, 1993
5. Diaz et al., J. Agric. Food Chem., 52 : 4562-4659, 2004
6. Garna et al., J. Agric. Food Chem., 52 : 4562-4659, 2004
7. Combo et al., Biotechnol. Agron. Soc. Environ., 15 : 153-164, 2011
8. Demande JP 2012187042
9. Demande EP 2308989
10. Demande KR 20130070396
11. Demande UA 54330
12. Demande GB 792518
13. Demande US 2014134310
14. Laurent et Boulenguer, Food Hydrocoll., 17 : 445-454, 2003
15. Thakur et al., Crit. Rev. Food Sci. Nutr., 37 : 47-73, 1997
16. Mesbahi et al., Food Hydrocoll., 19 : 731-738, 2005
17. Courtois, Curr. Opin. Microbiol., 12 : 261-273, 2009
18. Wang, Food Res. Int., 42 : 8-12, 2009
19. Paulsen et Barsett, Adv. Polym. Sci., 186 : 69-101, 2005
20. Yamada et Kiyohara, Comprehensive Glycoscience, Elsevier, pp. 663-694, 2007
21. Chlubnova et al., Nat. Prod. Rep., 28 : 937-952, 2011
22. KR 20130070396
23. EP 2308989
24. KR 20130070526
25. US 3316242
26. JP 2007254403
27. Novosel'skaya et al., Chemistry of Natural Compounds, 36 : 1-10, 2000
28. Bertho et al., J. Chem. Soc. Chem. Commun., 1391-1393, 1995
29. Auvray et al., J. Mater. Chem., 7 : 1373-1376, 1997
30. Ferrières et al., Carbohydr. Res., 311 : 15-25, 1998
31. Benvegnu et al., Polym. Int., 52 : 500-506, 2003
32. Goodby et al., Chem. Soc. Rev., 36 : 1971-2032, 2007
33. Demande internationale WO 9302092
34. Demande FR 9402771
35. Wikiera et al., Food Chem., 172 : 675-680, 2015 (EPub 2014)

## Revendications

1. Procédé de préparation de compositions comprenant :
(i) des dérivés de l'acide D-galacturonique de formule (I) : 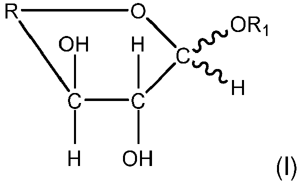 où
- R₁ est une chaine de 2 à 22 atomes de carbone linéaire ou ramifiée, saturée ou insaturée ;
- R est CH-CH(OH)-CO₂R₂ ou CH(OH)-CH-CO₂R₂, dont le carbone ne portant pas le groupe hydroxyle est lié à l'atome d'oxygène endocyclique ;
- R₂ est un hydrogène, R₁, un atome de métal alcalin, un atome de métal alcalino-terreux, ou un groupe ammonium quaternaire de formule (II) : 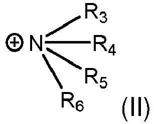 où chacun des R₃ à R₆ est indépendamment un atome d'hydrogène, un alkyle ayant de 1 à 6 atomes de carbone ou un hydroxyalkyle ayant de 1 à 6 atomes de carbone ; et
(ii) de 5 à 30% en masse de glycosides d'alkyle dérivés de sucres neutres ;
et **caractérisé en ce que** ledit procédé one pot comprend :
a) une étape d'hydrolyse acide complète d'une pectine ;
b) une étape d'estérification et glycosylation de l'hydrolysat issu de l'étape
a) par un alcool de formule R'OH, linéaire ou ramifié, saturé ou insaturé, ayant de 1 à 4 atomes de carbone ;
c) une étape de trans-estérification et trans-glycosylation de l'alkyl galactoside uronate d'alkyle issu de l'étape b) par un alcool de formule R"OH, linéaire ou ramifié, saturé ou insaturé, ayant de 2 à 22 atomes de carbone où R" est R₁.
d) et optionnellement une étape de saponification de l'ester issu de l'étape c).

2. Procédé selon la revendication 1, où l'étape d'hydrolyse a) est réalisée en présence d'eau et/ou d'un solvant ionique et d'un catalyseur acide, de préférence à une température de 80°C à 120°C.

3. Procédé selon la revendication 2, où le solvant ionique est choisi dans le groupe constitué de : chlorure de 1-butyl-3-méthylimidazolium, bromure de 1-butyl-3-méthylimidazolium, méthylsulfate de tris-(2-hydroxyéthyl)méthylammonium, et acétate de 1-éthyl-3-méthylimidazolium.

4. Procédé selon la revendication 2, où le catalyseur acide est choisi dans le groupe constitué de : l'acide chlorhydrique, l'acide sulfurique, un acide alkyl sulfurique, un acide sulfonique, un acide alkylsulfonique ou un sulfosuccinate d'alkyl, les acides perhalohydriques, des métaux, leurs oxydes ou leurs sels comme leurs halogénures.

5. Procédé selon la revendication 4, où le catalyseur chimique est l'acide méthylsulfonique.

6. Procédé selon l'une quelconque des revendications 1 à 5, où l'alcool R'OH est le n-butanol.

7. Procédé selon l'une quelconque des revendications 1 à 6, où l'alcool R"OH est choisi dans le groupe constitué des alcools gras linéaires, saturés ou insaturés.

8. Procédé selon l'une quelconque des revendications 1 à 7, où l'étape d'estérification et glycosylation b) est conduite à la pression atmosphérique et à la température d'ébullition de l'eau ou de l'azéotrope formé avec l'alcool R'OH.

9. Procédé selon l'une quelconque des revendications 1 à 8, où l'étape de trans-estérification et trans-glycosylation c) est réalisée à 70°C sous pression réduite afin de recycler l'alcool R'OH.

10. Procédé de préparation de sels d'acides alkyl galactosides uroniques selon le procédé tel que défini dans l'une quelconque des revendications 1 à 9 et comprenant en outre une étape d) de saponification de l'ester issu de l'étape c).

11. Procédé selon la revendication 10, où l'étape de saponification d) est réalisée en présence (i) d'une base M(OH)x dans laquelle M est un métal alcalin ou alcalino-terreux, et x est la valence, ou (ii) d'une base 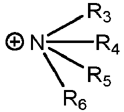 où chacun des R₃ à R₆ est indépendamment un atome d'hydrogène, un alkyle ayant de 1 à 6 atomes de carbone ou un hydroxyalkyle ayant de 1 à 6 atomes de carbone.

12. Procédé selon la revendication 11, où la base est choisie dans le groupe constitué de : l'hydroxyde de sodium, l'hydroxyde de potassium, l'ammoniaque ou un hydroxyde d'alkyl(hydroxyalkyl)ammonium.

13. Procédé selon l'une quelconque des revendications 10 à 12, où l'étape de saponification d) est réalisée à une température de 0°C à 100°C.

14. Procédé de préparation d'acides alkyl galactoside uroniques selon le procédé défini dans l'une quelconque des revendications 10 à 13 et comprenant en outre une étape e) d'acidification des sels issus de l'étape d).

15. Procédé selon la revendication 14, où l'étape d'acidification e) est réalisée en présence d'un acide choisi dans le groupe constitué de : l'acide chlorhydrique, l'acide sulfurique, un acide sulfonique ou une résine sulfonique sous sa forme H⁺. l'étape de saponification d) est réalisée à une température de 0°C à 100°C.

16. Composition obtenue par un procédé selon l'une quelconque des revendications 1 à 15 à partir d'un hydrolysat de pectine, **caractérisée en ce que** ladite composition présente une teneur en glycosides d'alkyles dérivés de sucres neutres, comprise entre 5 et 30 % en masse.

17. Composition selon la revendication 16, où la teneur en glycosides d'alkyles dérivés de sucres neutres est de 5 % en masse.

18. Utilisation d'une composition selon la revendication 16 ou 17 comme agent tensioactif.

19. Utilisation d'une composition selon la revendication 18, où l'agent tensioactif est choisi dans le groupe constitué des émulsifiants et des détergents.

## Patentansprüche

1. Verfahren zur Herstellung von Zusammensetzungen, umfassend:
(i) D-Galakturonsäurederivate der Formel (I): 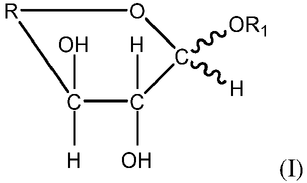 in welcher
- R₁ eine lineare oder verzweigte, gesättigte oder ungesättigte Kette mit 2 bis 22 Kohlenstoffatomen ist;
- R ist CH-CH(OH)-CO₂R₂ oder CH(OH)-CH-CO₂R₂, wobei der Kohlenstoff, der die Hydroxylgruppe nicht trägt, an das endocyclische Sauerstoffatom gebunden ist;
- R₂ ein Wasserstoff, R₁, ein Alkalimetallatom, ein Erdalkalimetallatom oder eine quaternäre Ammoniumgruppe der Formel (II) ist: 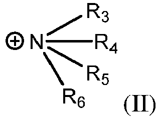 in der jeder der Reste R₃ bis R₆ unabhängig ein Wasserstoffatom, ein Alkyl mit 1 bis 6 Kohlenstoffatomen oder ein Hydroxyalkyl mit 1 bis 6 Kohlenstoffatomen ist; und
(ii) von 5 bis 30 Gew.-% Alkylglycoside, die von neutralen Zuckern abgeleitet sind; und **dadurch gekennzeichnet, dass** das Ein-Topf-Verfahren umfasst:
a) einen Schritt der sauren Hydrolyse eines Pektins;
b) einen Schritt der Veresterung und Glykosylierung des aus Schritt a) resultierenden Hydrolysats mit einem linearen oder verzweigten, gesättigten oder ungesättigten Alkohol der Formel R'OH mit 1 bis 4 Kohlenstoffatomen;
c) einen Schritt der Umesterung und Transglykosylierung des aus Schritt b) resultierenden Alkyl(alkylgalaktosid)uronats mit einem linearen oder verzweigten, gesättigten oder ungesättigten Alkohol der Formel R"OH mit 2 bis 22 Kohlenstoffatomen, in der R'' R₁ ist;
d) und gegebenenfalls einen Schritt der Verseifung des aus Schritt c) resultierenden Esters.

2. Verfahren nach Anspruch 1, wobei der Hydrolyseschritt a) in Gegenwart von Wasser und/oder einem ionischen Lösungsmittel und einem Säurekatalysator, vorzugsweise bei einer Temperatur von 80°C bis 120°C, durchgeführt wird.

3. Verfahren nach Anspruch 2, wobei das ionische Lösungsmittel ausgewählt ist aus der Gruppe bestehend aus: 1-Butyl-3-methylimidazoliumchlorid, 1-Butyl-3-methylimidazoliumbromid, Tris-(2-hydroxyethyl)methylammoniummethylsulfat und 1-Ethyl-3-methylimidazoliumacetat.

4. Verfahren nach Anspruch 2, wobei der Säurekatalysator ausgewählt ist aus der Gruppe bestehend aus: Salzsäure, Schwefelsäure, einer Alkylschwefelsäure, einer Sulfonsäure, einer Alkylsulfonsäure oder einem Alkylsulfosuccinat, perhalogenierten Säuren, Metallen, den Oxiden oder den Salzen davon, wie den Halogeniden davon.

5. Verfahren nach Anspruch 4, wobei der chemische Katalysator Methylsulfonsäure ist.

6. Verfahren nach einem der Ansprüche 1 bis 5, wobei der Alkohol R'OH n-Butanol ist.

7. Verfahren nach einem der Ansprüche 1 bis 6, wobei der Alkohol R"OH ausgewählt ist aus der Gruppe bestehend aus linearen, gesättigten oder ungesättigten Fettalkoholen.

8. Verfahren nach einem der Ansprüche 1 bis 7, wobei der Schritt der Veresterung und Glykosylierung b) bei Atmosphärendruck und bei dem Siedepunkt von Wasser oder des mit dem Alkohol R'OH gebildeten Azeotrops durchgeführt wird.

9. Verfahren nach einem der Ansprüche 1 bis 8, wobei der Umesterungs- und Transglykosylierungsschritt c) bei 70°C unter vermindertem Druck durchgeführt wird, um den Alkohol R'OH zu recyceln.

10. Verfahren zur Herstellung von Salzen von Alkylgalactosiduronsäuren gemäß dem Verfahren nach einem der Ansprüche 1 bis 9, das ferner einen Schritt d) der Verseifung des aus Schritt c) resultierenden Esters umfaßt.

11. Verfahren nach Anspruch 10, wobei der Verseifungsschritt d) in Gegenwart von (i) einer Base M(OH)x, in der M ein Alkali-oder Erdalkalimetall und x die Wertigkeit ist, oder (ii) einer 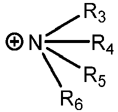 Base, in der jeder der Reste R₃ bis R₆ unabhängig voneinander ein Wasserstoffatom, ein Alkyl mit 1 bis 6 Kohlenstoffatomen oder ein Hydroxyalkyl mit 1 bis 6 Kohlenstoffatomen ist, durchgeführt wird.

12. Verfahren nach Anspruch 11, wobei die Base ausgewählt ist aus der Gruppe bestehend aus: Natriumhydroxid, Kaliumhydroxid, wässrigem Ammoniak oder einem Alkyl(hydroxyalkyl)ammoniumhydroxid.

13. Verfahren nach einem der Ansprüche 10 bis 12, wobei der Verseifungsschritt d) bei einer Temperatur von 0°C bis 100°C durchgeführt wird.

14. Verfahren zur Herstellung von Alkylgalactosiduronsäuren gemäß dem Verfahren nach einem der Ansprüche 10 bis 13 und ferner einen Schritt e) des Ansäuerns der aus Schritt d) resultierenden Salze umfassend.

15. Verfahren nach Anspruch 14, wobei der Ansäuerungsschritt e) in Gegenwart einer Säure, ausgewählt aus der Gruppe, bestehend aus: Salzsäure, Schwefelsäure, einer Sulfonsäure oder einem Sulfonsäureharz in seiner H+-Form, durchgeführt wird, der Verseifungsschritt d) bei einer Temperatur von 0°C bis 100°C durchgeführt wird.

16. Zusammensetzung, die nach einem Verfahren gemäß einem der Ansprüche 1 bis 15 aus einem Pektinhydrolysat erhalten wird, **dadurch gekennzeichnet, daß** die Zusammensetzung einen Gehalt an von neutralen Zuckern abgeleiteten Alkylglycosiden zwischen 5 und 30 Gew.-% aufweist.

17. Zusammensetzung nach Anspruch 16, wobei der Gehalt an von neutralen Zuckern abgeleiteten Alkylglykosiden 5 Gew.-% beträgt.

18. Verwendung einer Zusammensetzung nach Anspruch 16 oder 17 als Tensid.

19. Verwendung einer Zusammensetzung nach Anspruch 18, wobei das Tensid ausgewählt ist aus der Gruppe bestehend aus Emulgatoren und Detergenzien.

## Claims

1. Method for preparing compositions comprising:
(i) D-galacturonic acid derivatives of formula (I): 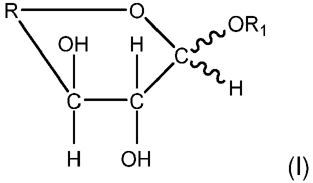 in which
- R₁ is a linear or branched, saturated or unsaturated chain of 2 to 22 carbon atoms;
- R is CH-CH(OH)-CO₂R₂ or CH(OH)-CH-CO₂R₂, in which the carbon not bearing the hydroxyl group is bonded to the endocyclic oxygen atom;
- R₂ is a hydrogen, R₁, an alkali metal atom, an alkaline earth metal atom or a quaternary ammonium group of formula (II): 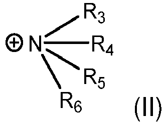 in which each of R₃ to R₆ is independently a hydrogen atom, an alkyl having from 1 to 6 carbon atoms or a hydroxyalkyl having from 1 to 6 carbon atoms; and
(ii) from 5 to 30% by weight of alkyl glycosides derived from neutral sugars; and **characterized in that** said one pot method comprises:
a) a step of complete acid hydrolysis of a pectin;
b) a step of esterification and glycosylation of the hydrolyzate resulting from step a) with a linear or branched, saturated or unsaturated alcohol of formula R'OH having from 1 to 4 carbon atoms;
c) a step of transesterification and transglycosylation of the alkyl(alkyl galactosid)uronate resulting from step b) with a linear or branched, saturated or unsaturated alcohol of formula R"OH having from 2 to 22 carbon atoms in which R" is R₁;
d) and optionally a step of saponification of the ester resulting from step c).

2. Method as claimed in claim 1, wherein the hydrolysis step a) is carried out in the presence of water and/or an ionic solvent and an acid catalyst, preferably at a temperature of 80°C to 120°C.

3. Method as claimed in claim 2, wherein the ionic solvent is selected from the group consisting of: 1-butyl-3-methylimidazolium chloride, 1-butyl-3-methylimidazolium bromide, tris-(2-hydroxyethyl)methylammonium methylsulfate, and 1-ethyl-3-methylimidazolium acetate.

4. Method as claimed in claim 2, wherein the acid catalyst is selected from the group consisting of: hydrochloric acid, sulfuric acid, an alkylsulfuric acid, a sulfonic acid, an alkylsulfonic acid or an alkylsulfosuccinate, perhalohydric acids, metals, the oxides or the salts thereof, such as the halides thereof.

5. Method as claimed in claim 4, wherein the chemical catalyst is methylsulfonic acid.

6. Method as claimed in any one of claims 1 to 5, wherein the alcohol R'OH is n-butanol.

7. Method as claimed in any one of claims 1 to 6, wherein the alcohol R"OH is selected from the group consisting of linear, saturated or unsaturated fatty alcohols.

8. Method as claimed in any one of claims 1 to 7, wherein the step of esterification and glycosylation b) is carried out at atmospheric pressure and at the boiling point of water or of the azeotrope formed with the alcohol R'OH.

9. Method as claimed in any one of claims 1 to 8, wherein the transesterification and transglycosylation step c) is carried out at 70°C under reduced pressure in order to recycle the alcohol R'OH.

10. Method for preparing salts of alkyl galactoside uronic acids according to the method as defined in any one of claims 1 to 9 and further comprising a step d) of saponification of the ester resulting from step c).

11. Method as claimed in claim 10, wherein the saponification step d) is carried out in the presence of (i) a base M(OH)x, in which M is an alkali metal or alkaline earth metal and x is the valency, or (ii) a base 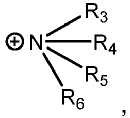 in which each of R₃ to R₆ is independently a hydrogen atom, an alkyl having from 1 to 6 carbon atoms or a hydroxyalkyl having from 1 to 6 carbon atoms.

12. Method as claimed in claim 11, wherein the base is selected from the group consisting of: sodium hydroxide, potassium hydroxide, aqueous ammonia or an alkyl(hydroxyalkyl)ammonium hydroxide.

13. Method as claimed in any one of claims 10 to 12, wherein the saponification step d) is carried out at a temperature of 0°C to 100°C.

14. Method for preparing alkyl galactoside uronic acids according to the method defined in any one of claims 10 to 13 and further comprising a step e) of acidification of the salts resulting from step d).

15. Method as claimed in claim 14, wherein the acidification step e) is carried out in the presence of an acid selected from the group consisting of: hydrochloric acid, sulfuric acid, a sulfonic acid or a sulfonic resin in its H⁺ form. the saponification step d) is carried out at a temperature of 0°C to 100°C.

16. Composition obtained by a method as claimed in any one of claims 1 to 15 from a pectin hydrolyzate, **characterized in that** said composition has a content of alkyl glycosides derived from neutral sugars, of between 5 and 30% by weight.

17. Composition as claimed in claim 16, wherein the content of alkyl glycosides derived from neutral sugars is 5% by weight.

18. Use of a composition as claimed in claim 16 or 17 as surfactant.

19. Use of a composition as claimed in claim 18, wherein the surfactant is selected from the group consisting of emulsifiers and detergents.
